# EUROPEAN PATENT SPECIFICATION

(11) **EP 2 255 790 B1**
(45) Date of publication and mention of the grant of the patent: **06.12.2017**
(21) Application number: 10164044.9
(22) Date of filing: 27.05.2010
(51) Int. Cl.: A61K 31/192, A61K 9/127

(54) **Liposome composition comprising naproxen, and a method of obtaining same**
Liposomzusammensetzung enthaltend Naproxen und herstellungsverfahren dafür
Composition liposomique comprenant du Naproxen et son procédé d'obtention

(30) Priority: 28.05.2009 PL 38813409
(43) Date of publication of application: 01.12.2010
(73) Proprietor: Przedsiebiorstwo Produkcji Farmaceutycznej Hasco-Lek S.A., 51-131 Wroclaw (PL)
(72) Inventor: Borowik, Tomasz, 50-439, Wroclaw (PL); Langner, Marek, 51-314, Wroclaw (PL); Przybylo, Magdalena, 51-165, Wroclaw (PL); Potaczek, Piotr, 58-508, Jelenia Gora (PL)
(74) Representative: Krekora, Magdalena

(56) References cited:
- EP-A2- 1 815 846
- WO-A1-01/60336
- WO-A1-2004/032900
- WO-A1-2008/114274
- US-A1- 2003 235 610
- R. VALJAKKA-KOSKELA ET AL: "Enhancement of percutaneous absorption of naproxen by phospholipids" INTERNATIONAL JOURNAL OF PHARMACEUTICS, vol. 175, December 1998 (1998-12), pages 225-230, XP002593115
- FLATEN G E ET AL: "Drug permeability across a phospholipid vesicle-based barrier" EUROPEAN JOURNAL OF PHARMACEUTICAL SCIENCES, ELSEVIER, AMSTERDAM, NL LNKD- DOI:10.1016/J.EJPS.2008.04.001, vol. 34, no. 2-3, 3 July 2008 (2008-07-03) , pages 173-180, XP022696128 ISSN: 0928-0987 [retrieved on 2008-04-15]

## Description

### TECHNICAL FIELD

The object of the present invention is a liposome composition comprising naproxen and excipients used for medical or cosmetic purposes, and a method of obtaining a liposome composition comprising naproxen and excipients.

### BACKGROUND ART

Naproxen is a well-known active substance belonging to a group of nonsteroidal anti-inflammatory drugs (NSAIDs) commonly used for the reduction of inflammation, pain, and fever. It works by inhibiting cyclooxygenases (COX-1, COX-2), taking part in prostaglandins synthesis. Medicines comprising Naproxen are mostly administered orally or topically. Because of its chemical structure naproxen is hardly soluble in aqueous solvents. But only dissolved (monomeric) naproxen is active and effective in skin permeation, when applied topically.

One of the methods to enhance naproxen solubility in aqueous solvents is to transform it into the form of salt, preferably sodium salt. However topical skin preparations comprising naproxen sodium as active substance have lower pharmaceutical efficacy in comparison to preparations comprising naproxen in its free form. It is a result of lower concentration of active substance, as naproxen sodium salt has strong surface activity.

Another method of enhancing the amount of dissolved fractions of naproxen is to use organic solvents such as alcohols (ethanol) or diols (propylene glycol). For instance patent application EP0872247 refers to gels comprising naproxen, buffer, ethanol and propylene glycol as solvent, and gelling agents. Another patent application EP1014942 describes formulations comprising from 50 to 85% of ethanol, isopropanol or mixture thereof as solvents. However use of alcohol in high concentrations as a base for topical skin formulations creates a significant risk because of their dehydrating and irritant properties.

Another method of adjusting solubility of naproxen is to provide microemulsions based on two-phase system - oil-in-water type. An example of such a solution is provided in patent application EP 1249231, where naproxen is incorporated into microemulsion comprising mixture of triglycerides, fatty acids, and a surfactant.

Other known solutions are based on the concept of closing hardly soluble compounds in aggregates of polymer compounds (e.g. WO9915165) or in lipid-liposome aggregates (WO2004032900) in order to enhance their transdermal diffusion. The invention is also based on this concept. The patent application WO2004032900 describes use of highly deformable lipid vesicles for effective transportation of NSAIDs, including naproxen, through skin. Highly deformable vesicles are formed by at least three amphipatic substances including active substance and a surfactant, and have ability to penetrate skin and stay intact.

Experimental data published by Valjakka-Koskela et al. (International Journal of Pharmaceuticals 175 (1998) 225-230) is the closest prior art to the present invention. The authors state, that use of combination of propylene glycol, as co-solvent, and phospholipids increases transdermal penetration of naproxen in system *in vitro.* The authors observe increase of transdermal penetration of naproxen in compositions where the weight ratio of propylene glycol to lipid is from 80:1 to 26.7:1. The authors observe that further increase of phospholipid amount does not result in increase of transdermal penetration value of naproxen. From the description samples preparation is known that examined formulations were multilayer structures having closely undefined sizes (uncalibrated aggregates). Similarly literature data (Nishihata et al., Chem. Pharm. Bull. (1987) 35: 3807-3812) on diclofenac (NSAID) show that increase of content of phospholipids (hydrogenated soya lecithin) above 0.5% by weight does not affect the value of transdermal flux of the compound.

### DISCLOSURE OFTHE INVENTION

The objective of the present invention is to provide a liposome composition comprising naproxen, having increased, in comparison to known compositions, value of transdermal diffusion of active substance resulting in higher efficacy of pharmaceutical substance *in vivo,* by use of lipid carriers in combination with co-solvent selected from the group of double alcohols and appropriate, pharmaceutically acceptable, excipients. Additionally, use of lipid carriers increases naproxen photostability in compositions being the subject of the invention.

The invention describes a liposome composition comprising naproxen and excipients, characterised in that it comprises 15 - 25% by weight of phospholipids in conjunction with propylene glycol, in mass proportion of phospholipid to propylene glycol from 0.6:1 to 1.67:1, and wherein the calibrated aggregates have hydrodynamic diameter from 50 to 500 nm determined by method of dynamic dispersion of light. The liposome composition may comprise about 10% by weight of free acid naproxen or of a mixture of free acid naproxen and pharmaceutically acceptable naproxen salts, preferably sodium salt of naproxen. The phospholipid may be phosphatidylcholine, for instance soya phosphatidylcholine. The liposome composition according to the invention preferably comprises excipients such as: antioxidant, preserving agent, buffering agents, local blood perfusion-enhancing agents, and gelling agent. Excipients used in the composition are: the antioxidant - disodium edetate, preserving agent - methyl parahydroxybenzoate, buffering substances - phosphatic salts, local blood circulation enhancing agent - chloral hydrate, gelling agent - ammonium acryloyldimethyltaurate/VP copolymer.
The excipients in the liposome composition may be used in following amounts: 0-0.5% by weight of disodium edetate, 0-0.5% by weight of methyl parahydroxybenzoate, 0-1.5% by weight of phosphatic salts, 0-0.3% by weight of chloral hydrate, and 0-1% by weight of ammonium acryloyldimethyltaurate/VP copolymer. The value of pH of the liposome composition is in the range from 4 to 7.5, preferably 5 - 6.5. The liposome composition according to the invention is in the form of gel - a finished medical preparation for topical use.

A method of obtaining the liposome composition comprising naproxen and excipients, described in the invention, consist in that naproxen and excipients are dissolved in a phospholipid solution having concentration of 15-25% by weight, with propylene glycol as a solvent in mass proportion of phospholipid to diol from 0.6:1 to 1.67:1, where the quantity of naproxen and excipients is such to enable calibration of multilayer lipid structures obtained after hydratation. The multilayer lipid structures, obtained as a result of dispersing the lipid solution in the aqueous phase, are calibrated. They may be calibrated by extrusion through polycarbonate filters of dimension 100 - 400 nm. Another possibility is to calibrate the multilayer lipid structures by homogenisation.

Use of extrusion or homogenisation allows for reduction of multilayerdness of obtained structures, as well as reduction of their size distribution width. The obtained liposome gel, comprising calibrated liposomes encapsulating active substance, is then complemented with the amount of missing active substance, in accordance with the recipe, and substances enhancing local blood circulation. Additionally, in order to enhance density of the composition, a density enhancer may be added during the process of production. Alternatively the whole amount of active substance is added after calibration of multilayer liposomes.

Size of liposome structures obtained during the process of manufacturing was determined for 50 - 500 nm by method of dynamic dispersion of light. Range of pH of obtained formulations was determined for 4 - 7.5, preferably 5 - 6.5.

Because of low naproxen solubility in aqueous solvents and amphipatic character of molecule, use of lipid carrier in liposome form, in combination with appropriately chosen co-solvent and excipients allows for obtaining of preparation having increased amount of naproxen in dissolved form. The tests that were carried out on solubility of naproxen in complex macromolecular systems showed drastic decrease of naproxen solubility in aqueous solutions of propylene glycol in comparison to solubility in pure glycol. Moreover it was shown that presence of liposomes in aqueous solutions increases its solubility.

Unexpectedly it was discovered that this solubility, in a system based on equal amounts by weight of propylene glycol and liposomes, increases four times in proportion to its value in a system comprising propylene glycol and liposomes in weight ratio 10 to 1 or higher (Diagram 1).

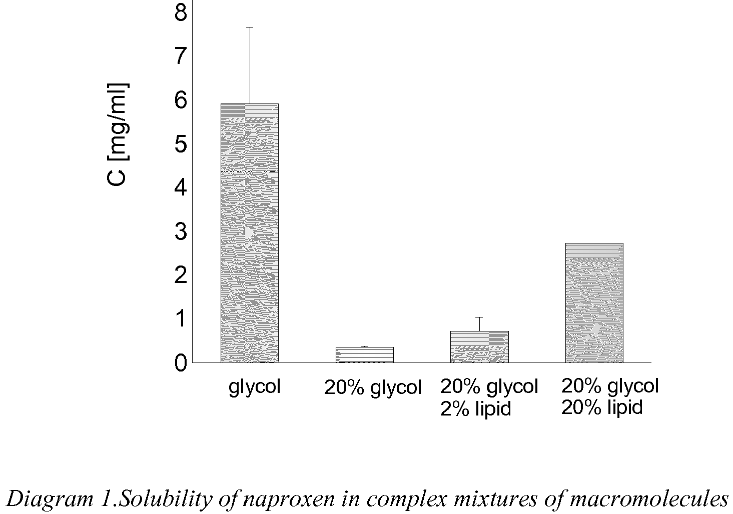

The inventors observed that use of phospholipids in amounts 15-25% by weight, in form of calibrated aggregates in conjunction with propylene glycol in mass ration between 0.6:1 and 1.67:1 increases by about two times the flux of transdermal diffusion of naproxen in relation to formulations comprising small amounts of phospholipid (2% by weight). Comparison of experimental data is shown in Table 1. Tests of transdermal diffusion *in vitro* in Franz cells on pig skin showed that use of liposome carrier in combination with appropriate amount of propylene glycol increases by two times the percentage of transdermal diffusion of active substance in relation to placebo not comprising liposomes (Diagram 2). Compositions 1-3 are comparative examples. Table 1

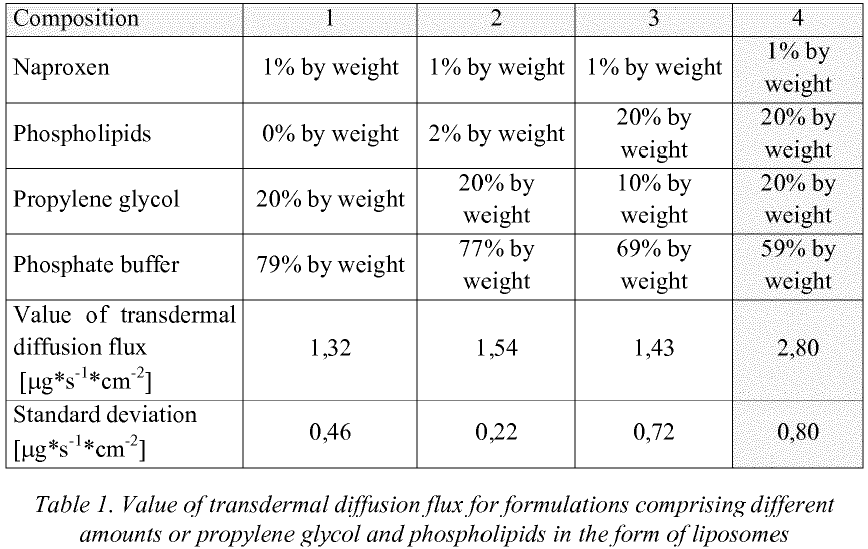

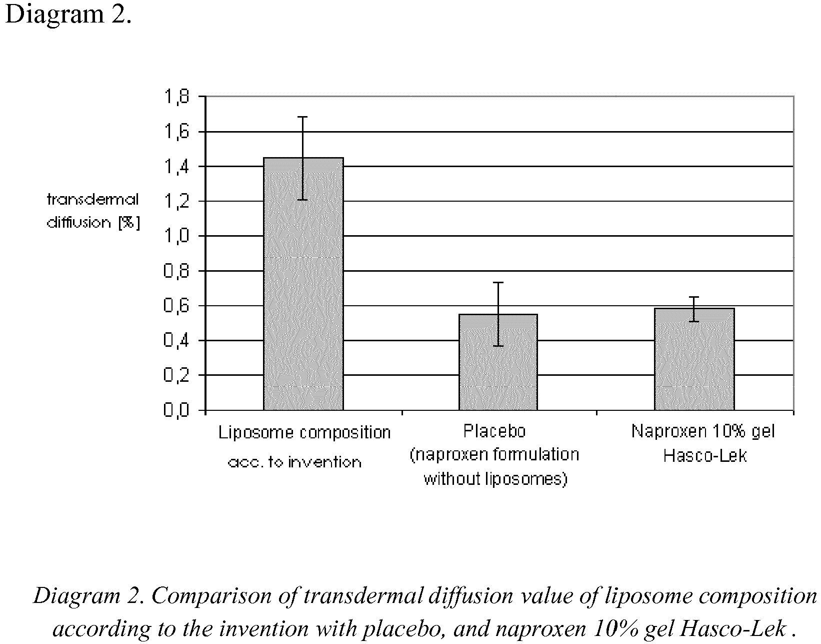

Additionally, use of liposomes as carriers of active substances improves organoleptic parameters of obtained topical skin preparations, such as angle of adhesion of the formulation or its smearing.

The experiments proved that naproxen and its salts (sodium salt) undergo photodegradation in aqueous solutions. Instability of active substance caused by external factors creates a significant danger with regard to stability of the whole pharmaceutical composition. In order to obtain information on this subject, time-distribution spectra of emission from naproxen were collected in the range between 340 and 500 nm (time interval between spectrum maxima - 72 seconds) with induction wavelength of 330 nm (the farthest induction maximum in the infrared direction). Collected time-distribution spectra show modification in naproxen molecule illuminated by wavelength of 330 nm in minute time scale. Evident decrease of main emission maximum intensity at 360 nm, corresponding to decrease of naproxen molecules in the solution, is accompanied by appearance of second maximum at wavelength of 440 nm, being a result of light emission from naproxen photodisintegration product (Diagram 3).

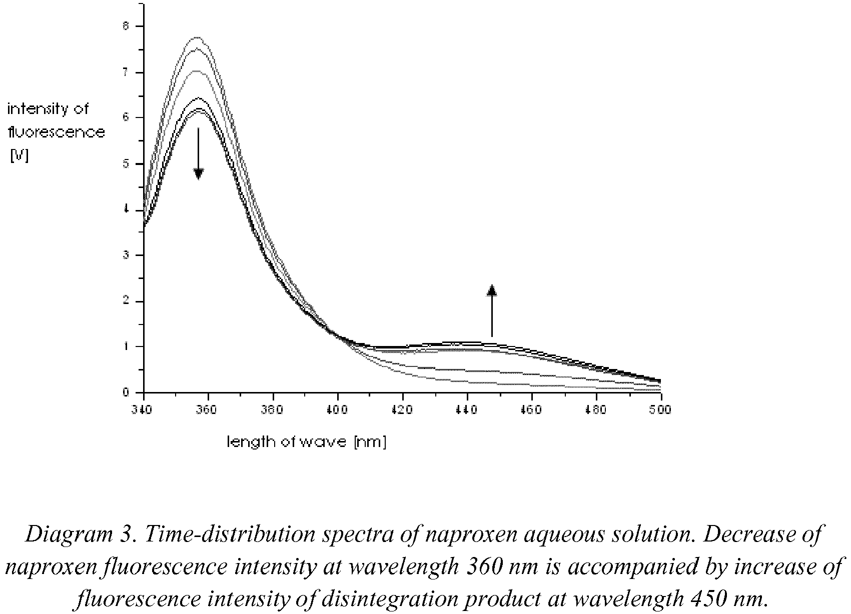

It was unexpectedly observed that presence of lipid in the form of calibrated liposomes, in weight ratio 2:1 in proportion to naproxen, increased naproxen photostability by two times (Diagram 4).

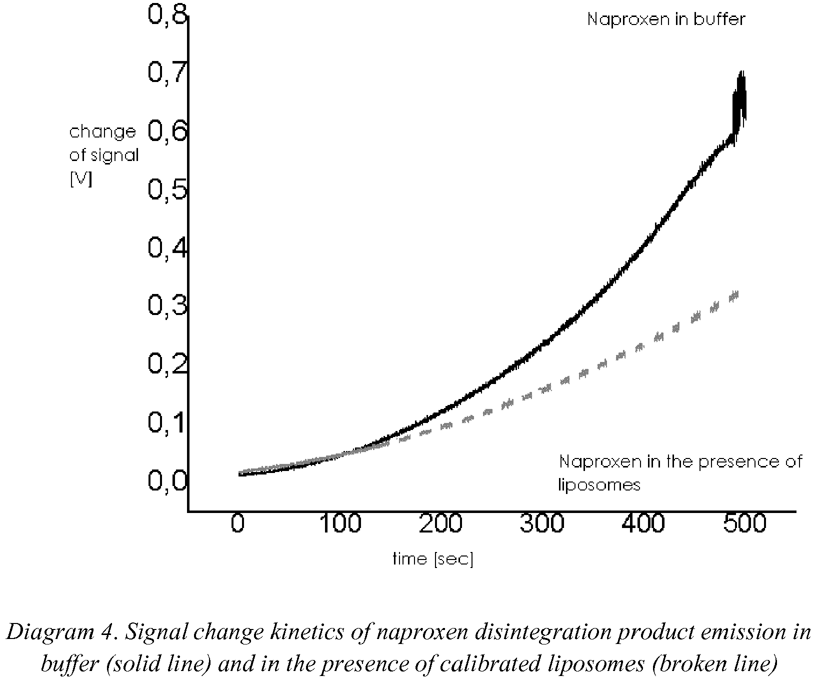

### BEST MODE OF CARRYING OUT THE INVENTION

The following examples illustrate the invention without setting or delineating its limits.

### Example 1.

Purified phosphatidylcholine and propylene glycol are poured into the agitator tank equipped with heating jacket and vacuum pump, and mixed at temperature of 70°C, under vacuum, until total dissolution of phosphatidylcholine. To the obtained solution of purified phosphatidylcholine are added: disodium edetate and methyl parahydroxybenzoate. Then the entirety, when deaerating, is dispergated in de-ionized water until intimate mixture of multilayer liposomes is obtained.

Deaerated and warmed to 70°C suspension is extruded once at room temperature through two series connected polycarbonate filters of pore dimension 100 nm (Nucleopore, Whatman). Intermediate product - calibrated liposomes - are collected to the tank for intermediate product. Every time the size of obtained structures is measured by dynamic light dispersion method (Zetasizer Nano S, Malvern Instruments).

Appropriate amount of obtained suspension of big monolayer liposomes is transferred to agitator tank. Suspension of liposomes is mixed at room temperature under vacuum, when adding in sequence: naproxen, and chloral hydrate.

To the deaerated intermediate product is added, when mixing, ammonium acryloyldimethyltaurate/VP copolymer. Finished product is deaerated. The composition of obtained formulation is shown in the table. Comparison of the product transdermal diffusion in relation to naproxen Hasco-Lek gel 10% is shown at Diagram 5.

| **Substance** | **Dose** [mg/lg] |
|---|---|
| Naproxen | 100,0 |
| Purified soya phosphatidylcholine | 180,0 |
| Propylene glycol | 180,0 |
| Methyl parahydroxybenzoate | 1,5 |
| Chloral hydrate | 2,0 |
| Disodium edetate | 1,5 |
| Ammonium acryloyldimethyltaurate/VP copolymer | 5,0 |
| Purified water | 530,0 |
| ∑ | 1000,0 |

### Qualitative-quantitative composition of formulation No. 1.

### Example 2.

Purified phosphatidylcholine and propylene glycol are poured into the agitator tank equipped with heating jacket and vacuum pump, and mixed at temperature of 70°C, under vacuum, until total dissolution of phosphatidylcholine. To the obtained solution of purified phosphatidylcholine are added: naproxen, disodium edetate and methyl parahydroxybenzoate. Then the entirety, when deaerating, is dispergated in phosphate buffer until intimate mixture of multilayer liposomes is obtained.

Deaerated and warmed to 70°C suspension is extruded once at room temperature through two series connected polycarbonate filters of pore dimension 100 nm (Nucleopore, Whatman). Intermediate product - calibrated liposomes - are collected to the tank for intermediate product. Every time the size of obtained structures is measured by dynamic light dispersion method (Zetasizer Nano S, Malvern Instruments).

Appropriate amount of obtained suspension of big monolayer liposomes is transferred to agitator tank. Suspension of liposomes is mixed at room temperature under vacuum, when adding in sequence: naproxen, and chloral hydrate.

To the deaerated intermediate product is added, when mixing, ammonium acryloyldimethyltaurate/VP copolymer. Finished product is deaerated. The composition of obtained formulation is shown in the table. Comparison of the product transdermal diffusion in relation to naproxen Hasco-Lek gel 10% is shown at Diagram 5.

| **Substance** | **Dose** [mg/1g] |
|---|---|
| Naproxen | 100,0 |
| Purified soya phosphatidylcholine | 180,0 |
| Propylene glycol | 180,0 |
| Methyl parahydroxybenzoate | 1,5 |
| Chloral hydrate | 2,0 |
| Disodium edetate | 1,5 |
| Sodium dihydrogen phosphate dihydrate | 0,3 |
| Sodium dihydrogen phosphate anhydride | 0,4 |
| Ammonium acryloyldimethyltaurate/VP copolymer | 5,0 |
| Purified water | 529,3 |
| ∑ | 1000,0 |

### Qualitative-quantitative composition of formulation No. 2.

### Example 3.

Purified phosphatidylcholine and propylene glycol are poured into the agitator tank equipped with heating jacket and vacuum pump, and mixed at temperature of 70°C, under vacuum, until total dissolution of phosphatidylcholine. To the obtained solution of purified phosphatidylcholine are added: naproxen and ethanol. Then the entirety, when deaerating, is dispergated in phosphate buffer until intimate mixture of multilayer liposomes is obtained.

Deaerated and warmed to 70°C suspension is extruded once at room temperature through two series connected polycarbonate filters of pore dimension 100 nm (Nucleopore, Whatman). Intermediate product - calibrated liposomes - are collected to the tank for intermediate product. Every time the size of obtained structures is measured by dynamic light dispersion method (Zetasizer Nano S, Malvern Instruments).

Appropriate amount of obtained suspension of big monolayer liposomes is transferred to agitator tank. Suspension of liposomes is mixed at room temperature under vacuum, when adding in sequence: naproxen, and chloral hydrate.

To the deaerated intermediate product is added, when mixing, ammonium acryloyldimethyltaurate/VP copolymer. Finished product is deaerated. The composition of obtained formulation is shown in the table. Comparison of the product transdermal diffusion in relation to naproxen Hasco-Lek gel 10% is shown at Diagram 5.

| **Substance** | **Dose** [mg/1g] |
|---|---|
| Naproxen | 100,0 |
| Purified soya phosphatidylcholine | 180,0 |
| Propylene glycol | 180,0 |
| Ethanol | 50,0 |
| Chloral hydrate | 2,0 |
| Sodium dihydrogen phosphate dihydrate | 0,3 |
| Sodium dihydrogen phosphate anhydride | 0,4 |
| Ammonium acryloyldimethyltaurate/VP copolymer | 5,0 |
| Purified water | 482,3 |
| ∑ | 1000,0 |

### Qualitative-quantitative composition of formulation No. 3.

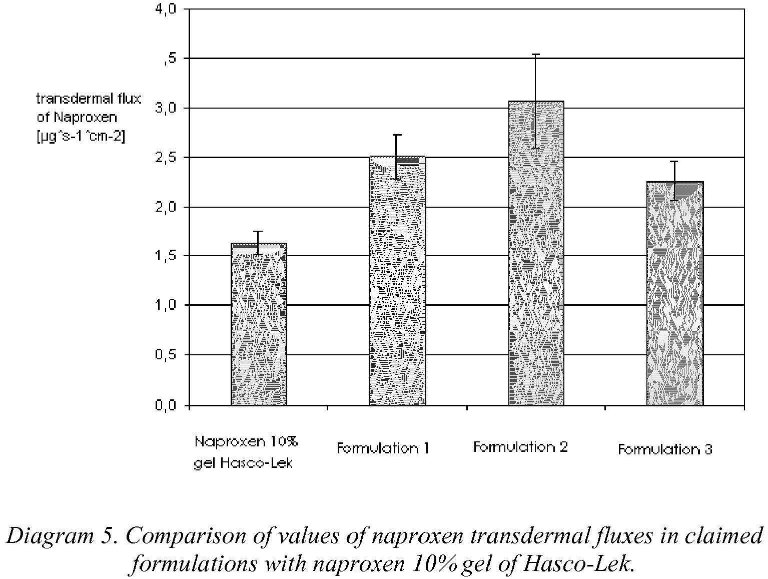

### Example 4.

Purified phosphatidylcholine and propylene glycol are poured into the agitator tank equipped with heating jacket and vacuum pump, and mixed at temperature of 70°C, under vacuum, until total dissolution of phosphatidylcholine. To the obtained solution of purified phosphatidylcholine are added in sequence: lavender aromatic composition, naproxen, disodium edetate, and methyl parahydroxybenzoate. Then the entirety, when deaerating, is dispergated in phosphate buffer until intimate mixture of multilayer liposomes is obtained.

Deaerated and warmed to 70°C suspension is extruded once at room temperature through a polycarbonate filter of pore dimension 100 nm (Nucleopore, Whatman). Intermediate product - calibrated liposomes - are collected to the tank for intermediate product. Every time the size of obtained structures is measured by dynamic light dispersion method (Zetasizer Nano S, Malvern Instruments).

Appropriate amount of obtained suspension of big monolayer liposomes is transferred to agitator tank. Suspension of liposomes is mixed at room temperature under vacuum, when adding in sequence: naproxen, and chloral hydrate. To the deaerated intermediate product is added, when mixing, ammonium acryloyldimethyltaurate/VP copolymer. Finished product is deaerated. The composition of obtained formulation is shown in the table.

| **Substance** | **Dose** [mg/1g] |
|---|---|
| Naproxen | 100,0 |
| Purified soya phosphatidylcholine | 180,0 |
| Propylene glycol | 180,0 |
| Methyl parahydroxybenzoate | 1,5 |
| Chloral hydrate | 2,0 |
| Disodium edetate | 1,5 |
| Sodium dihydrogen phosphate dihydrate | 0,3 |
| Sodium dihydrogen phosphate anhydride | 8,7 |
| Ammonium acryloyldimethyltaurate/VP copolymer | 5,0 |
| Lavender aromatic composition | 11,8 |
| Purified water | 509,2 |
| ∑ | 1000,0 |

### Qualitative-quantitative composition of formulation No. 4.

### Example 5.

Purified phosphatidylcholine and propylene glycol are poured into the agitator tank equipped with heating jacket and vacuum pump, and mixed at temperature of 70°C, under vacuum, until total dissolution of phosphatidylcholine. To the obtained solution of purified phosphatidylcholine are added in sequence: naproxen, disodium edetate, and methyl parahydroxybenzoate. Then the entirety, when deaerating, is dispergated in phosphate buffer until intimate mixture of multilayer liposomes is obtained.

Deaerated and warmed to 70°C suspension is homogenised at room temperature until liposomes of about 100 nm are obtained. Intermediate product - calibrated liposomes - are collected to the tank for intermediate product. Every time the size of obtained structures is measured by dynamic light dispersion method (Zetasizer Nano S, Malvern Instruments).

Appropriate amount of obtained suspension of big monolayer liposomes is transferred to agitator tank. Suspension of liposomes is mixed at room temperature under vacuum, when adding in sequence: naproxen, and chloral hydrate. To the deaerated intermediate product is added, when mixing, ammonium acryloyldimethyltaurate/VP copolymer. Finished product is deaerated. The composition of obtained formulation is shown in the table.

| **Substance** | **Dose** [mg/1g] |
|---|---|
| Naproxen | 100,0 |
| Purified soya phosphatidylcholine | 180,0 |
| Propylene glycol | 180,0 |
| Methyl parahydroxybenzoate | 1,5 |
| Chloral hydrate | 2,0 |
| Disodium edetate | 1,5 |
| Sodium dihydrogen phosphate dihydrate | 0,3 |
| Sodium dihydrogen phosphate anhydride | 0,4 |
| Ammonium acryloyldimethyltaurate/VP copolymer | 5,0 |
| Purified water | 529,3 |
| ∑ | 1000,0 |

### Qualitative-quantitative composition of formulation No. 5.

### Example 6.

Purified phosphatidylcholine and propylene glycol are poured into the agitator tank equipped with heating jacket and vacuum pump, and mixed at temperature of 70°C, under vacuum, until total dissolution of phosphatidylcholine. To the obtained solution of purified phosphatidylcholine are added in sequence: naproxen, and ethanol. Then the entirety, when deaerating, is dispergated in phosphate buffer until intimate mixture of multilayer liposomes is obtained.

Deaerated and warmed to 70°C suspension is homogenised at room temperature until liposomes of about 100 nm are obtained. Intermediate product - calibrated liposomes - are collected to the tank for intermediate product. Every time the size of obtained structures is measured by dynamic light dispersion method (Zetasizer Nano S, Malvern Instruments).

Appropriate amount of obtained suspension of big monolayer liposomes is transferred to agitator tank. Suspension of liposomes is mixed at room temperature under vacuum, when adding in sequence: naproxen, and chloral hydrate. To the deaerated intermediate product is added, when mixing, ammonium acryloyldimethyltaurate/VP copolymer. Finished product is deaerated. The composition of obtained formulation is shown in the table.

| **Substance** | **Dose** [mg/1g] |
|---|---|
| Naproxen | 100,0 |
| Purified soya phosphatidylcholine | 180,0 |
| Propylene glycol | 180,0 |
| Ethanol | 50,0 |
| Chloral hydrate | 2,0 |
| Sodium dihydrogen phosphate dihydrate | 0,3 |
| Sodium dihydrogen phosphate anhydride | 0,4 |
| Ammonium acryloyldimethyltaurate/VP copolymer | 5,0 |
| Purified water | 482,3 |
| ∑ | 1000,0 |

*Qualitative-quantitative composition of formulation No. 6.*

## Claims

1. A liposome composition comprising naproxen and excipients, **characterised in that** it comprises 15 - 25% by weight of phospholipids in the form of calibrated aggregates in conjunction with propylene glycol, in mass proportion of phospholipid to propylene glycol from 0.6:1 to 1.67:1, and wherein the calibrated aggregates have hydrodynamic diameter from 50 to 500 nm determined by method of dynamic dispersion of light.

2. The liposome composition according to claim 1, **characterised in that** it comprises about 10% by weight of free acid naproxen or of a mixture of free acid naproxen and pharmaceutically acceptable naproxen salts.

3. The liposome composition according to any of the preceding claims, **characterised in that** the phospholipid is phosphatidylcholine.

4. The liposome composition according to any of the preceding claims, **characterised in that** it comprises antioxidant, preserving agent, buffering agents, local blood circulation enhancing agents, and gelling agent.

5. The liposome composition according to any of the preceding claims, **characterised in that** the antioxidant is disodium edetate, preserving agent is methyl parahydroxybenzoate, buffering substances are phosphatic salts, local blood circulation enhancing agent is chloral hydrate, gelling agent is ammonium acryloyldimethyltaurate/VP copolymer.

6. The liposome composition according to claim 5, **characterised in that** it comprises 0-0.5% be weight of disodium edetate, 0-0.5% by weight of methyl parahydroxybenzoate, 0-1.5% by weight of phosphatic salts, 0-0.3% by weight of chloral hydrate, and 0-1% by weight of ammonium acryloyldimethyltaurate/VP copolymer.

7. The liposome composition according to any of the preceding claims, **characterised in that** it has pH in the range from 4 to 7.5, preferably 5 -6.5.

8. The liposome composition according to any of the preceding claims, **characterised in that** it is in the form of gel.

9. A method of obtaining the liposome composition comprising naproxen and excipients as defined in claims from 1 to 8, **characterised in that** naproxen and excipients are dissolved in a phospholipid solution with propylene glycol as a solvent, where the quantity of naproxen and excipients is such to enable calibration of multilayer lipid structures obtained after hydratation as a result of dispersing the lipid solution in the aqueous phase, and where those multilayer lipid structures are calibrated to obtain hydrodynamic diameter from 50 to 500 nm determined by method of dynamic dispersion of light.

10. The method according to claim 9, **characterised in that** multilayer lipid structures, obtained as a result of dispersing the lipid solution in the aqueous phase, are calibrated by extrusion through polycarbonate filters of dimension 100 - 400 nm.

11. The method according to claim 9, **characterised in that** multilayer lipid structures, obtained as a result of dispersing the lipid solution in the aqueous phase, are calibrated by homogenisation.

## Patentansprüche

1. Liposomzusammensetzung, enthaltend Naproxen und Hilfsstoffe, **dadurch gekennzeichnet, dass** sie 15 bis 25 Gew.-% Phospholipide in Form von kalibrierten Aggregaten im Zusammenhang mit Propylenglykol enthält, in einem Massenverhältnis von Phospholipid zu Propylenglycol von 0,6:1 bis 1,67:1, und worin die kalibrierten Aggregate einen hydrodynamischen Durchmesser von 50 bis 500 nm haben, ermittelt durch das Verfahren der dynamischen Dispersion von Licht.

2. Liposomzusammensetzung nach Anspruch 1, **dadurch gekennzeichnet, dass** sie ca. 10 Gew.-% freies Säurennaproxen enthält, oder einer Mischung aus freiem Säurennaproxen und pharmazeutisch akzeptablen Naproxensalzen.

3. Liposomzusammensetzung nach irgendeinem der vorstehenden Ansprüche, **dadurch gekennzeichnet, dass** das Phospholipid ein Phosphatidylcholin ist.

4. Liposomzusammensetzung nach irgendeinem der vorstehenden Ansprüche, **dadurch gekennzeichnet, dass** sie Antioxidans, Konservierungsmittel, Puffermittel, lokale durchblutungsfördernde Mittel und Geliermittel enthält.

5. Liposomzusammensetzung nach irgendeinem der vorstehenden Ansprüche, **dadurch gekennzeichnet, dass** das Antioxidans Dinatriumedetat ist, das Konservierungsmittel Methylparahydroxybenzoat, die Puffermittel phosphathaltige Salze, das lokale durchblutungsfördernde Mittel Chloralhydrat und das Geliermittel Ammoniumacryloyldimethyltaurat/VP-Copolymer ist.

6. Liposomzusammensetzung nach Anspruch 5, **dadurch gekennzeichnet, dass** sie 0 bis 0,5 Gew.-% Dinatriumedetat, 0 bis 0,5 Gew.% Methylparahydroxybenzoat, 0 bis 1,5 Gew.-% phosphathaltige Salze, 0 bis 0,3 Gew.-% Chloralhydrat und 0 bis 1 Gew.-% Ammoniumacryloyldimethyltaurat/ VP-Copolymer enthält.

7. Liposomzusammensetzung nach irgendeinem der vorstehenden Ansprüche, **dadurch gekennzeichnet, dass** sie einen pH-Wert im Bereich von 4 bis 7,5, vorzugsweise von 5 bis 6,5 aufweist.

8. Liposomzusammensetzung nach irgendeinem der vorstehenden Ansprüche, **dadurch gekennzeichnet, dass** sie in Form eines Gels vorliegt.

9. Herstellungsverfahren für Liposomzusammensetzung, enthaltend Naproxen und Hilfsstoffe, wie in den Ansprüchen 1 bis 8 definiert, **dadurch gekennzeichnet, dass** Naproxen und die Hilfsstoffe in einer Phospholipidlösung mit Propylenglykol als Lösungsmittel gelöst sind, wobei die Menge an Naproxen und Hilfsstoffen dergestalt ist, dass eine Kalibrierung von mehrschichtigen Lipidstrukturen, die nach der Hydratation infolge einer Dispersion der Lipidlösung in der wässrigen Phase erhalten wurden, möglich ist, und wobei jene mehrschichtigen Lipidstrukturen so kalibriert sind, dass ein hydrodynamischer Durchmesser von 50 bis 500 nm, ermittelt durch das Verfahren der dynamischen Dispersion von Licht, erhalten wird.

10. Verfahren nach Anspruch 9, **dadurch gekennzeichnet, dass** mehrschichtige Lipidstrukturen, die durch die Dispersion der Lipidlösung in der wässrigen Phase erhalten wurden, durch Extrusion durch Polycarbonatfilter mit dem Maß 100 bis 400 nm kalibriert werden.

11. Verfahren nach Anspruch 9, **dadurch gekennzeichnet, dass** mehrschichtige Lipidstrukturen, die durch die Dispersion der Lipidlösung in der wässrigen Phase erhalten wurden, durch Homogenisierung kalibriert werden.

## Revendications

1. Composition liposomique comprenant du Naproxen et des excipients, **caractérisée en ce qu'**elle comprend de 15 à 25 % en poids de phospholipides sous la forme d'agrégats calibrés conjointement avec du propylène glycol, en une proportion massique du phospholipide au propylène glycol de 0,6/1 à 1,67/1, et dans laquelle les agrégats calibrés ont un diamètre hydrodynamique de 50 à 500 nm déterminé par le procédé de dispersion dynamique de la lumière.

2. Composition liposomique selon la revendication 1, **caractérisée en ce qu'**elle comprend environ 10 % en poids de Naproxen acide libre ou d'un mélange de Naproxen acide libre et de sels de Naproxen pharmaceutiquement acceptables.

3. Composition liposomique selon l'une quelconque des revendications précédentes, **caractérisée en ce que** le phospholipide est de la phosphatidylcholine.

4. Composition liposomique selon l'une quelconque des revendications précédentes, **caractérisée en ce qu'**elle comprend un antioxydant, un agent de conservation, des agents tampons, des agents d'amélioration de la circulation sanguine locale et un agent gélifiant.

5. Composition liposomique selon l'une quelconque des revendications précédentes, **caractérisée en ce que** l'antioxydant est de l'édétate de disodium, l'agent de conservation est du parahydroxybenzoate de méthyle, les substances tampons sont des sels phosphatiques, l'agent d'amélioration de la circulation sanguine locale est un hydrate de chloral, l'agent gélifiant est un copolymère d'acryloyldiméthyltaurate d'ammonium/VP.

6. Composition liposomique selon la revendication 5, **caractérisée en ce qu'**elle comprend de 0 à 0,5 % en poids d'édétate de disodium, de 0 à 0,5 % en poids de parahydroxybenzoate de méthyle, de 0 à 1,5 % en poids de sels phosphatiques, de 0 à 0,3 % en poids d'hydrate de chloral et de 0 à 1 % en poids de copolymère d'acryloyldiméthyltaurate d'ammonium/VP.

7. Composition liposomique selon l'une quelconque des revendications précédentes, **caractérisée en ce qu'**elle a un pH dans la plage de 4 à 7,5, de préférence de 5 à 6,5.

8. Composition liposomique selon l'une quelconque des revendications précédentes, **caractérisée en ce qu'**elle se présente sous la forme d'un gel.

9. Procédé d'obtention de la composition liposomique comprenant du Naproxen et des excipients selon les revendications 1 à 8, **caractérisé en ce que** le Naproxen et les excipients sont dissous dans une solution de phospholipides avec du propylène glycol en tant que solvant, où la quantité de Naproxen et d'excipients est adaptée pour permettre la calibration de structures lipidiques multicouches obtenues après hydratation en résultat de la dispersion de la solution de lipides dans la phase aqueuse, et où ces structures lipidiques multicouches sont calibrées pour obtenir un diamètre hydrodynamique de 50 à 500 nm déterminé par le procédé de dispersion dynamique de la lumière.

10. Procédé selon la revendication 9, **caractérisé en ce que** les structures lipidiques multicouches, obtenues en résultat de la dispersion de la solution de lipides dans la phase aqueuse, sont calibrées par extrusion à travers des filtres en polycarbonate d'une dimension de 100 à 400 nm.

11. Procédé selon la revendication 9, **caractérisé en ce que** les structures lipidiques multicouches, obtenues en résultat de la dispersion de la solution de lipides dans la phase aqueuse, sont calibrées par homogénéisation.
